# FASCICULE DE BREVET EUROPEEN

(11) **EP 3 373 859 B1**
(45) Date de publication et mention de la délivrance du brevet: **05.07.2023**
(21) Numéro de dépôt: 15825814.5
(22) Date de dépôt: 12.11.2015
(51) Int. Cl.: A61F 2/24

(54) **PROTHÈSE DE VALVE CARDIAQUE MITRALE OU TRICUSPIDE**
PROTHETISCHE MITRAL- ODER TRIKUSPIDALHERZKLAPPE
MITRAL OR TRICUSPID HEART VALVE PROSTHESIS

(43) Date de publication de la demande: 19.09.2018
(73) Titulaire: Valmy Holding, 59650 Villeneuve d'Ascq (FR)
(72) Inventeur: MODINE, Thomas, 59110 La Madeleine (FR)
(74) Mandataire: LLR
(86) Numéro de dépôt international: PCT/IB2015/058754
(87) Numéro de publication internationale: WO 2017/081516

(56) Documents cités:
- EP-A1- 2 478 868
- WO-A1-2009/132187
- WO-A1-2011/051043
- WO-A1-2013/037519
- WO-A1-2015/052663
- FR-A1- 3 021 209

## Description

La présente invention concerne une prothèse de valve cardiaque mitrale ou tricuspide.

Une pathologie bien connue d'une valve cardiaque, touchant principalement les patients âgés, est la distension de l'anneau valvulaire, cette distension conduisant à une mauvaise coaptation des valvules et donc à la perte d'étanchéité et d'efficacité de la valve.

Il est connu de traiter cette pathologie par annuloplastie, c'est-à-dire par la mise en place d'un anneau prothétique total ou partiel ayant pour but de re-calibrer l'anneau valvulaire natif. Cette technique a certains inconvénients, notamment de ne pas être très adaptée à des patients âgés.

Il est également connu d'implanter, par-dessus la valve native, une prothèse comprenant une armature tubulaire expansible et une valve prothétique montée sur cette armature. L'armature tubulaire, fréquemment dénommée "stent", a une structure maillée, et comprend une portion atriale, c'est-à-dire destinée à prendre place au niveau de l'oreillette d'un coeur, une portion ventriculaire, c'est-à-dire destinée à prendre place au niveau du ventricule d'un coeur, et une portion annulaire, c'est-à-dire destinée à prendre place au niveau de l'anneau valvulaire natif, située entre ces portions atriale et ventriculaire. L'armature peut être auto-expansible (étant notamment en un matériau à mémoire de forme) ou en un matériau expansible au moyen d'un ballonnet. La valve prothétique peut, quant à elle, être en un matériau synthétique ou naturel.

Une telle prothèse est déformable de manière à pouvoir être placée dans un cathéter et est destinée à être déployée depuis ce cathéter au niveau de la valve native à traiter. Elle peut être mise en place par la voie d'abord apicale (c'est-à-dire par la pointe inférieure du coeur) ou par une voie transeptale (valve mitrale) ou jugulaire ou fémorale (valve tricuspide).

Les prothèses de ce type existantes ne donnent pas parfaitement satisfaction, présentant notamment des risques de migration et d'obstruction de la voie d'éjection ventriculaire, ayant une mise en place pas toujours très aisée à opérer, n'ayant pas une forme parfaitement adaptée à celle du site d'implantation, et impliquant d'aménager un orifice d'accès au site d'implantation de diamètre relativement important, et donc relativement traumatique.

WO11/051043 présente une prothèse de valve cardiaque comprenant une portion atriale formée d'arcs et une portion annulaire maillée.

La présente invention a pour objectif principal de remédier à ces inconvénients essentiels. Elle a notamment pour objectif de fournir une prothèse apte à être introduite dans un coeur par un orifice ayant un diamètre de l'ordre de 18 mm, contre 22 mm avec les prothèses existantes.

La prothèse concernée est du type précité et comprend les caractéristiques de la revendication 1.

La prothèse selon l'invention comprend ainsi une structure maillée uniquement au niveau de sa portion annulaire, qui, étant constituée de mailles en losange, est apte à prendre une forme radialement contractée de faible diamètre, inférieure à 18 mm à l'état de contraction, lorsque les filaments formant ces mailles sont à proximité immédiate les uns des autres ; ladite portion atriale et ladite portion ventriculaire ne sont pas formées par une structure maillée et, par contraction latérale des fils en U ou en V des extensions qui les constituent, sont également aptes à prendre une forme radialement contractée de relativement faible diamètre, inférieure à 18 mm à l'état de contraction.

La prothèse ainsi constituée est donc apte à être placée dans un cathéter ayant un diamètre externe de l'ordre de 18 mm, dont l'introduction est nettement moins traumatique pour la paroi cardiaque qu'un cathéter selon la technique antérieure.

Lorsque la prothèse est mise en place par la voie d'abord apicale, sa portion atriale est tout d'abord déployée et amenée contre la partie atriale de l'anneau valvulaire ; la forme sensiblement sphérique de cette portion atriale est bien adaptée à une large prise d'appui contre cette partie atriale de l'anneau et contre la paroi adjacente de l'oreillette ; ladite portion annulaire est ensuite déployée, ce qui amène les différentes pointes d'ancrage que comprend cette portion annulaire à venir s'insérer dans l'anneau valvulaire. La poursuite du recul du cathéter réalise le déploiement progressif de la portion ventriculaire ; cette portion, compte tenu de sa forme ovoïde, vient en application étroite contre la partie ventriculaire de l'anneau valvulaire et est bien adaptée à la forme du ventricule à proximité de cet anneau ; elle ne vient pas en conflit avec les structures anatomiques sous-jacentes. De plus, l'indépendance des extensions précitées formant cette portion ventriculaire permet à cette portion ventriculaire de ne présenter aucun risque d'appui excessif contre la paroi du ventricule au niveau de la valve aortique, susceptible de gêner le fonctionnement de cette valve aortique ou d'obstruer la chambre de chasse ventriculaire.

La prothèse ainsi structurée permet donc de parfaitement atteindre l'objectif précité de fournir une prothèse apte à être introduite dans un coeur par un orifice ayant un diamètre de l'ordre de 18 mm, tout en ayant des risques de migration faibles, une mise en place relativement aisée et une forme adaptée à celle du site d'implantation.

Il est à noter que la prothèse selon l'invention pourrait être implantée par une voie d'abord transeptale (valve mitrale) ou jugulaire ou fémorale (valve tricuspide), auquel cas la portion ventriculaire est déployée en premier et la portion atriale est déployée en deuxième.

De préférence, lesdites pointes d'ancrage sont sous la forme de griffes courbes se développant vers l'extérieur de ladite portion annulaire depuis leurs bases vers leurs extrémités libres pointues.

Les pointes d'ancrage ainsi conformées réalisent un ancrage efficace.

Les pointes d'ancrage sont de préférence présentes sur l'ensemble de la circonférence de ladite portion annulaire ; elles sont de préférence régulièrement réparties sur cette circonférence.

De préférence, les pointes d'ancrage sont disposées par paires, de telle sorte que l'une des pointes d'ancrage d'une paire soit située du côté atrial de la prothèse et fasse saillie vers le côté ventriculaire de cette prothèse et que l'autre pointe d'ancrage de cette même paire soit située du côté ventriculaire de la prothèse et fasse saillie vers le côté atrial de cette prothèse, les extrémités libres de ces pointes d'ancrage étant situées en regard l'une de l'autre, et les pointes d'ancrage d'une même paire étant courbes, avec leur côté concave sur l'intérieur de cette paire de telle sorte que les deux pointes d'ancrage d'une même paire de pointes présente la forme d'une "pince de crabe". Lors du déploiement de la prothèse avec utilisation de la voie d'abord apicale, les pointes d'ancrage atriales se déploient en premier, permettant d'accrocher le tissu annulaire, puis, dans un deuxième temps, au fur et à mesure de la progression du déploiement, les pointes d'ancrage ventriculaires se déploient à leur tour et permettent de parfaire l'ancrage de la prothèse, avec maintien des pointes d'ancrage en position intra-annulaire.

Cette de "pince de crabe" permet un ancrage très efficace de la prothèse à l'intérieur de l'anneau valvulaire natif. En effet, la structure proposée permet une implantation qui prend appui sur le tissu valvulaire péri-annulaire dans un premier temps (les pointes d'ancrage), qui est suivi par un appui complémentaire sur l'anneau valvulaire. Le système d'accroche en pince permet ainsi une accroche du tissu valvulaire et se complète par l'appui intra-annulaire de la structure métallique, et ce, en position mitrale et tricuspide quelque doit l'accès utilisé.

Ladite portion annulaire peut notamment comprendre six paires de pointes d'ancrage.

De préférence, les pointes d'ancrage ont leurs bases reliées aux portions des mailles qui forment les angles opposés de ces mailles dans la direction atriale-ventriculaire de la prothèse, ces pointes d'ancrage faisant saillie depuis ces portions des mailles.

Lors du déploiement de ladite portion annulaire, lesdits angles opposés des mailles se rapprochent l'un de l'autre, ce qui assure l'insertion des pointes d'ancrage dans l'anneau valvulaire, par fermeture de la pince que forme chaque paire de pointes d'ancrage.

Avantageusement, l'armature comprend, du côté de la base des extensions ventriculaires, des entretoises en forme de V reliant une portion de fil latéral d'une extension à la portion de fil latéral de l'extension adjacente.

Ces entretoises assurent le bon déploiement des extensions ventriculaires et permettent une rigidité légèrement renforcée de l'armature au niveau de la base des extensions ventriculaires, sans pour autant gêner l'aptitude de cette armature à être contractée radialement.

L'invention sera bien comprise, et d'autres caractéristiques et avantages de celle-ci apparaîtront, en référence au dessin schématique annexé, qui représente, à titre d'exemple non limitatif, une forme de réalisation préférée de la prothèse concernée.
La figure 1 en est une vue latérale, légèrement décalée vers le côté atrial de cette prothèse ;
la figure 2a est une vue agrandie d'une portion de la prothèse telle que montrée sur la figure 1, comprenant des griffes d'ancrage ;
la figure 2b est une vue de cette même portion, en coupe, selon un angle perpendiculaire à la vue selon la figure 2a ;
la figure 3 est une vue de la prothèse par son extrémité ventriculaire, légèrement désaxée, avec la valve prothétique que comprend cette prothèse en position d'ouverture ;
la figure 4 en est une vue par son extrémité atrial, avec la valve prothétique en position d'ouverture, et

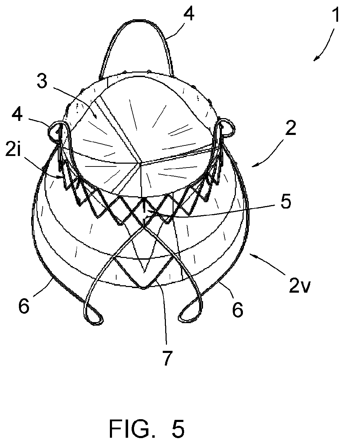

la figure 5 en est une vue similaire à la figure 4, avec la valve prothétique en position de fermeture.

Les figures représentent une prothèse 1 de valve cardiaque mitrale ou tricuspide, comprenant une armature tubulaire expansible 2 et une valve prothétique 3 montée sur cette armature.

L'armature 2 a structure formée de filaments élastiquement déformables, lui permettant de prendre la forme déployée représentée sur les figures et une forme contractée dans laquelle elle peut être contenue dans un cathéter (non représenté), d'introduction dans un coeur. Elle peut notamment être réalisée, par une technique connue, en alliage à mémoire de forme, notamment de nickel et de titane connu sous la dénomination Nitinol.

L'armature 2 comprend une portion atriale 2a, c'est-à-dire destinée à prendre place au niveau de l'oreillette d'un coeur, une portion ventriculaire 2v, c'est-à-dire destinée à prendre place au niveau du ventricule d'un coeur, et une portion annulaire intermédiaire 2i, située entre ces portions atriale 2a et ventriculaire 2v, destinée à prendre place au niveau de l'anneau valvulaire natif.

La portion atriale 2a est formée de trois extensions 4 en fil métallique, régulièrement réparties sur sa circonférence et se projetant depuis ladite portion annulaire 2i. Chaque extension 4 est formée d'un fil unique, en U ou V inversé, se développant selon une forme courbe. A l'état d'expansion de la prothèse 1, représentée sur les figures, la base de chaque extension 4 s'oriente radialement vers l'extérieur de la portion annulaire 2i puis l'extension 4 se recourbe vers l'intérieur de l'armature 2, de telle sorte que les trois extensions 4 soient sensiblement inscrites dans une sphère tronquée imaginaire placée au-dessus de ladite portion annulaire 2i.

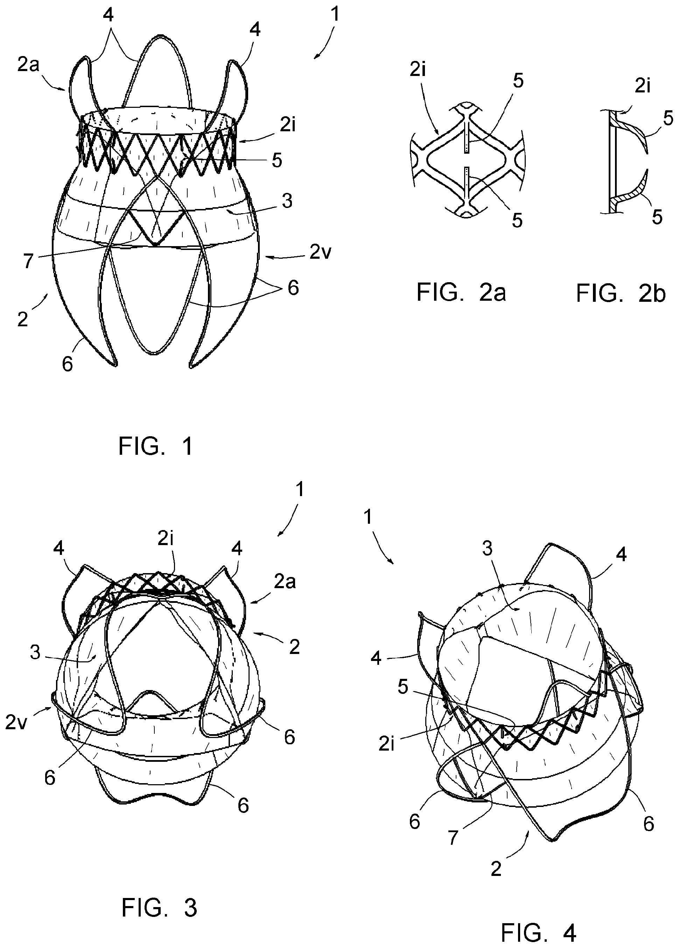

La portion annulaire 2i est formée de mailles en losange reliées les unes aux autres par leurs angles, et comprend six paires de griffes d'ancrage 5 faisant saillie de sa face extérieure, régulièrement réparties sur son pourtour. Comme cela est plus particulièrement visible sur les figures 2a et 2b, une griffe 5 d'une paire de griffes a sa base reliée à la portion de la maille qui forme l'angle de la maille situé sur le côté atrial de la prothèse 1, et l'autre griffe 5 de cette même paire de griffes a sa base reliée à la portion de la maille qui forme l'angle de la maille situé sur le côté ventriculaire de la prothèse, et les deux griffes 5 font saillie depuis ces portions de la maille, l'une vers l'autre ; ces griffes 5 de chaque paire de griffes sont courbes, avec leur côté concave sur l'intérieur de cette paire, de telle sorte que les deux griffes 5 forment une "pince de crabe" , de manière à former une sorte de pince. Les griffes 5 sont courbes et se développent vers l'extérieur de la prothèse 1 depuis ces bases vers leurs extrémités libres pointues.

La portion ventriculaire 2v est formée de trois extensions 6 en fil métallique, régulièrement réparties sur sa circonférence et se projetant depuis ladite portion annulaire 2i. Chaque extension 6 est formée d'un fil unique, en U ou V inversé, se développant selon une forme courbe. A l'état d'expansion de la prothèse, la base de chaque extension 6 s'oriente radialement vers l'extérieur de la portion annulaire 2i puis l'extension 6 se recourbe vers l'intérieur de l'armature 2 de telle sorte que les trois extensions 6 soient sensiblement inscrites dans une forme ovoïde imaginaire placée au-dessous de ladite portion annulaire 2i.

L'armature 2 comprend en outre, du côté de la base des extensions 6, des entretoises 7 en forme de V reliant une portion de fil latéral d'une extension 6 à la portion de fil latéral de l'extension 6 adjacente.

La valve prothétique 2, quant à elle est de type connu, réalisée à partir d'un matériau synthétique ou naturel (tel que du péricarde de porc), et ayant trois valvules dans l'exemple représenté.

En pratique, la prothèse 1 est contractée et placée dans un cathéter d'introduction dans un coeur, qui peut être introduit dans un coeur par la voie d'abord apicale. Du fait qu'elle comprend une structure maillée uniquement au niveau de sa portion annulaire 2i, constituée de mailles en losange, et du fait de la structure précitée des portions atriale 1a et ventriculaire 2v, cette prothèse 1 est apte à prendre une forme contractée de faible diamètre, inférieure à 18 mm à l'état de contraction. Le cathéter peut donc avoir un diamètre externe de l'ordre de 18 mm, dont l'introduction est nettement moins traumatique pour la paroi cardiaque qu'un cathéter selon la technique antérieure.

Lorsque la prothèse 1 est mise en place par la voie d'abord apicale, sa portion atriale 2a est tout d'abord déployée et amenée contre la partie atriale de l'anneau valvulaire ; la forme sensiblement sphérique de cette portion atriale 2a est bien adaptée à une large prise d'appui contre cette partie atriale de l'anneau et contre la paroi adjacente de l'oreillette ; ladite portion annulaire 2i est ensuite déployée, ce qui amène les différentes griffes 5 à s'insérer dans l'anneau valvulaire. Un bon pré-positionnement de la prothèse 1 vis-à-vis du site d'implantation est ainsi assuré.

La poursuite du recul du cathéter réalise le déploiement progressif de la portion ventriculaire 2v ; cette portion, compte tenu de sa forme ovoïde, vient en application étroite contre la partie ventriculaire de l'anneau valvulaire et est bien adaptée à la forme du ventricule à proximité de cet anneau ; elle ne vient pas en conflit avec les structures anatomiques sous-jacentes. De plus, l'indépendance des extensions 6 permet à la portion ventriculaire 2v de ne présenter aucun risque d'appui excessif contre la paroi du ventricule au droit de la valve aortique, susceptible de gêner le fonctionnement de cette valve aortique.

Il est à noter que la prothèse 1 pourrait être implantée par une voie d'abord transeptale (valve mitrale) ou jugulaire ou fémorale (valve tricuspide), auquel cas la portion ventriculaire 2v est déployée en premier et la portion atriale 2a est déployée en deuxième, avec des insertions des griffes 5 dans l'anneau s'opérant de la même manière que précitée.

L'invention fournit ainsi une prothèse de valve cardiaque mitrale ou tricuspide, présentant, par rapport aux prothèses homologues de la technique antérieure, les avantages déterminants de pouvoir être introduite dans un coeur par un orifice ayant un diamètre de l'ordre de 18 mm, de présenter des risques de migration très faibles, d'avoir une mise en place aisée à opérer, et d'avoir une forme adaptée au site d'implantation.

L'invention a été décrite ci-dessus en référence à une forme de réalisation donnée à titre d'exemple. Il va de soi qu'elle n'est pas limitée à cette forme de réalisation mais qu'elle s'étend à toutes les autres formes de réalisation couvertes par les revendications ci-annexées.

## Revendications

1. - Prothèse (1) de valve cardiaque mitrale ou tricuspide, comprenant une armature tubulaire expansible (2) et une valve prothétique (3) montée sur cette armature, ladite armature (2) comprenant une portion atriale (2a), destinée à prendre place au niveau d'une oreillette d'un coeur, une portion ventriculaire (2v) destinée à prendre place au niveau d'un ventricule du coeur, et une portion annulaire (2i) située entre ces portions atriale et ventriculaire et destinée à prendre place au niveau d'un anneau valvulaire du coeur ;
- ladite portion atriale (2a) est formée de trois ou quatre extensions (4) en fil métallique, régulièrement réparties sur la circonférence de cette portion atriale (2a) et se projetant depuis ladite portion annulaire (2i), chaque extension (4) étant formée d'un fil unique en U ou V inversé, **caractérisée en ce que**:
- les extensions se développant selon une forme courbe de manière telle qu'à l'état d'expansion de la prothèse (1), la base de cette extension s'oriente radialement vers l'extérieur de ladite portion annulaire (2i) puis que l'extension (4) se recourbe vers l'intérieur de l'armature (2), de telle sorte que les différentes extensions (4) soient sensiblement inscrites dans une sphère tronquée imaginaire placée au-dessus de ladite portion annulaire (2i) et soient agencées pour fournir une prise d'appui contre une partie atriale de l'anneau valvulaire et contre une paroi adjacente de l'oreillette ;
- ladite portion annulaire (2i) est formée de mailles en losange reliées les unes aux autres par leurs angles, qui comprennent des pointes d'ancrage (5) faisant saillie de sa face extérieure, dans lequel les pointes d'ancrage sont agencées pour être insérées dans l'anneau valvulaire ; et
- ladite portion ventriculaire (2v) est formée de trois ou quatre extensions (6) en fil métallique, régulièrement réparties sur la circonférence de cette portion ventriculaire (2v) et se projetant depuis ladite portion annulaire (2i), chaque extension étant formée d'un fil unique en U ou V et se développant selon une forme courbe de manière telle qu'à l'état d'expansion de la prothèse (1), la base de cette extension (6) s'oriente radialement vers l'extérieur de ladite portion annulaire (2i) puis que l'extension se recourbe vers l'intérieur de l'armature (2), de telle sorte que les différentes extensions (6) soient sensiblement inscrites dans une forme ovoïde imaginaire placée au-dessous de ladite portion annulaire (2i), et soient agencées pour venir en application contre une partie ventriculaire de l'anneau valvulaire et soient bien adaptées à la forme du ventricule.

2. - Prothèse (1) selon la revendication 1, **caractérisée en ce que** lesdites pointes d'ancrage sont sous la forme de griffes courbes (5) se développant vers l'extérieur de ladite portion annulaire (2i) depuis leurs bases vers leurs extrémités libres pointues.

3. - Prothèse (1) selon la revendication 1 ou la revendication 2, **caractérisée en ce que** les pointes d'ancrage (5) sont présentes sur l'ensemble de la circonférence de ladite portion annulaire (2i).

4. - Prothèse (1) selon l'une des revendications 1 à 3, **caractérisée en ce que** les pointes d'ancrage (5) sont régulièrement réparties sur la circonférence de ladite portion annulaire (2i).

5. - Prothèse (1) selon l'une des revendications 1 à 4, **caractérisée en ce que** les pointes d'ancrage (5) sont disposées par paires, de telle sorte que l'une des pointes d'ancrage d'une paire soit située du côté atrial de la prothèse (1) et fasse saillie vers le côté ventriculaire de cette prothèse (1) et que l'autre pointe d'ancrage de cette même paire soit située du côté ventriculaire de la prothèse (1) et fasse saillie vers le côté atrial de cette prothèse (1), les extrémités libres de ces pointes d'ancrage (5) étant situées en regard l'une de l'autre, et les pointes d'ancrage (5) d'une même paire sont courbes, avec leur côté concave sur l'intérieur de cette paire de telle sorte que les deux pointes d'ancrage (5) d'une même paire de pointes présente la forme d'une "pince de crabe".

6. - Prothèse (1) selon la revendication 5, **caractérisée en ce que** ladite portion annulaire (2i) comprend six paires de pointes d'ancrage (5).

7. - Prothèse (1) selon la revendication 5 ou la revendication 6, **caractérisée en ce que** les pointes d'ancrage (5) ont leurs bases reliées aux portions des mailles qui forment les angles opposés de ces mailles dans la direction atriale-ventriculaire de la prothèse (1), ces pointes d'ancrage faisant saillie depuis ces portions des mailles.

8. - Prothèse (1) selon l'une des revendications 1 à 7, **caractérisée en ce que** l'armature (2) comprend, du côté de la base des extensions ventriculaires (6), des entretoises (7) en forme de V reliant une portion de fil latéral d'une extension (6) à la portion de fil latéral de l'extension (6) adjacente.

## Patentansprüche

1. - Mitral- oder Trikuspidal-Herzklappenprothese (1), die ein dehnbares röhrenförmiges Gerüst (2) und eine auf dieser Armatur montierte Klappenprothese (3) aufweist, wobei das Gerüst (2) einen atrialen Abschnitt (2a) aufweist, der dazu bestimmt ist, einen Platz auf der Höhe eines Vorhofs eines Herzens einzunehmen, einen ventrikulären Abschnitt (2v), der dazu bestimmt ist, einen Platz an einem Ventrikel des Herzens einzunehmen, und einen ringförmigen Abschnitt (2i), der sich zwischen diesen atrialen und ventrikulären Abschnitten befindet und dazu bestimmt ist, einen Platz an einem Klappenring des Herzens einzunehmen;
- wobei der atriale Abschnitt (2a) aus drei oder vier Verlängerungen (4) aus Metalldraht gebildet ist, die gleichmäßig über den Umfang dieses atrialen Abschnitts (2a) verteilt sind und von dem ringförmigen Abschnitt (2i) vorstehen, wobei jede Verlängerung (4) aus einem einzelnen Draht in Form eines umgekehrten U oder V gebildet ist,
**dadurch gekennzeichnet, dass**:
- die Verlängerungen sich in einer gekrümmten Form erstrecken, so dass im ausgedehnten Zustand der Prothese (1) die Basis dieser Verlängerung sich radial nach außen des ringförmigen Abschnitts (2i) orientiert und die Verlängerung (4) sich dann zum Inneren des Gerüsts (2) hin krümmt, so dass die verschiedenen Verlängerungen (4) im Wesentlichen in eine imaginäre abgeschnittene Kugel eingeschrieben sind, die über dem ringförmigen Abschnitt (2i) angeordnet ist, und so angeordnet sind, dass sie einen Halt gegen einen atrialen Teil des Klappenrings und gegen eine angrenzende Wand des Vorhofs bieten;
- der ringförmige Abschnitt (2i) aus rautenförmigen Maschen gebildet ist, die an ihren Ecken miteinander verbunden sind und Verankerungsspitzen (5) aufweisen, die von ihrer Außenseite vorstehen, wobei die Verankerungsspitzen so angeordnet sind, dass sie in den Klappenring eingeführt werden können; und
- der ventrikuläre Abschnitt (2v) aus drei oder vier Verlängerungen (6) aus Metalldraht gebildet ist, die gleichmäßig über den Umfang dieses ventrikulären Abschnitt (2v) verteilt sind und von dem ringförmigen Abschnitt (2i) vorstehen, wobei jede Verlängerung aus einem einzelnen U- oder V-förmigen Draht gebildet ist und sich in einer gekrümmten Form erstreckt, so dass im ausgedehnten Zustand der Prothese (1) die Basis dieser Verlängerung (6) sich radial nach außen von dem ringförmigen Abschnitt (2i) orientiert und die Verlängerung sich dann zum Inneren des Gerüsts (2) hin krümmt, so dass die verschiedenen Verlängerungen (6) im Wesentlichen in eine imaginäre Eiform eingeschrieben sind, die unter dem ringförmigen Abschnitt (2i) angeordnet ist, und so angeordnet sind, dass sie an einem ventrikulären Teil des Klappenrings anliegen und gut an die Form des Ventrikels angepasst sind.

2. - Prothese (1) nach Anspruch 1, **dadurch gekennzeichnet, dass** die Verankerungsspitzen in Form von gekrümmten Krallen (5) vorliegen, die sich von ihren Basen zu ihren spitzen freien Enden nach außen von dem ringförmigen Abschnitt (2i) erstrecken.

3. - Prothese (1) nach Anspruch 1 oder Anspruch 2, **dadurch gekennzeichnet, dass** die Verankerungsspitzen (5) über den gesamten Umfang des ringförmigen Abschnitts (2i) vorhanden sind.

4. - Prothese (1) nach einem der Ansprüche 1 bis 3, **dadurch gekennzeichnet, dass** die Verankerungsspitzen (5) gleichmäßig über den Umfang des ringförmigen Abschnitts (2i) verteilt sind.

5. - Prothese (1) nach einem der Ansprüche 1 bis 4, **dadurch gekennzeichnet, dass** die Verankerungsspitzen (5) paarweise angeordnet sind, so dass eine der Verankerungsspitzen eines Paares auf der atrialen Seite der Prothese (1) liegt und zur ventrikulären Seite dieser Prothese (1) hin vorsteht und die andere Verankerungsspitze desselben Paares auf der ventrikulären Seite der Prothese (1) liegt und zur atrialen Seite dieser Prothese (1 ) hin vorsteht, die freien Enden dieser Verankerungsspitzen (5) einander gegenüberliegen und die Verankerungsspitzen (5) desselben Paares gekrümmt sind, wobei ihre Seite zur Innenseite dieses Paares hin konkav ist, so dass die beiden Verankerungsspitzen (5) desselben Paares von Spitzen die Form einer "Krabbenschere" aufweisen.

6. - Prothese (1) nach Anspruch 5, **dadurch gekennzeichnet, dass** der ringförmige Abschnitt (2i) sechs Paare von Verankerungsspitzen (5) aufweist.

7. - Prothese (1) nach Anspruch 5 oder 6, **dadurch gekennzeichnet, dass** die Verankerungsspitzen (5) mit ihren Basen mit den Abschnitten der Maschen verbunden sind, die die gegenüberliegenden Ecken dieser Maschen in der atrial-ventrikulären Richtung der Prothese (1) bilden, wobei die Verankerungsspitzen von diesen Abschnitten der Maschen vorstehen.

8. - Prothese (1) nach einem der Ansprüche 1 bis 7, **dadurch gekennzeichnet, dass** das Gerüst (2) auf der Seite der Basis der ventrikulären Verlängerungen (6) V-förmige Abstandshalter (7) aufweist, die einen Seitendrahtabschnitt einer Verlängerung (6) mit dem Seitendrahtabschnitt der benachbarten Verlängerung (6) verbinden.

## Claims

1. Mitral or tricuspid heart valve prosthesis (1), comprising an expandable tubular frame (2) and a prosthetic valve (3) mounted on the frame, said frame (2) comprising an atrial portion (2a), intended to be placed at the atrium of a heart, a ventricular portion (2v) intended to be placed at the ventricle of a heart, and an annular portion (2i) situated between these atrial and ventricular portions and intended to be placed at the native valve annulus;
- said atrial portion (2a) is made up of three or four metal-wire extensions (4), regularly distributed on the circumference of this atrial portion (2a) and projecting from said annular portion (2i), each extension (4) being made up of a single upside-down U-shaped or V-shaped wire,
**characterized in that**:
- extensions developing with a curved shape such that in the expansion state of the prosthesis (1), the base of this extension (4) is oriented radially toward the outside of said annular portion (2i), then the extension (4) curves toward the inside of the frame (2), such that the various extensions (4) are substantially part of an imaginary truncated sphere placed above said annular portion (2i) and are arranged to provide bearing against an atrial part of the valve annulus and against the adjacent wall of the atrium;
- said annular portion (2i) is made up of diamond meshes connected to one another by their corners, which comprise anchoring points (5) protruding from its outer face, in which the anchoring points are arranged to be inserted into the valve annulus; and
- said ventricular portion (2v) is made up of three or four metal-wire extensions (6), regularly distributed on the circumference of this ventricular portion (2v) and projecting from said annular portion (2i), each extension (6) being made up of a single U-shaped or V-shaped wire and developing with a curved shape such that in the expansion state of the prosthesis, the base of this extension (6) is oriented radially toward the outside of said annular portion (2i), then the extension curves toward the inside of the frame (2), such that the various extensions (6) are substantially part of an imaginary ovoid placed below said annular portion (2i), and are arranged to closely apply against the ventricular part of the valve annulus and are well suited to the shape of the ventricle.

2. Prosthesis (1) according to claim 1, **characterized in that** said anchoring points are in the form of curved claws (5) developing toward the outside of the said annular portion (2i) from their bases toward their pointed free ends.

3. Prosthesis (1) according to claim 1 or claim 2, **characterized in that** the anchoring points (5) are present on the entire circumference of said annular portion (2i).

4. Prosthesis (1) according to anyone of claims 1-3, **characterized in that** the anchoring points (5) are regularly distributed on the circumference of said annular portion (2i).

5. Prosthesis (1) according to anyone of claims 1-4, **characterized in that** the anchoring points (5) are arranged in pairs, such that one of the anchoring points of a pair is located on the atrial side of the prosthesis (1) and protrudes toward the ventricular side of this prosthesis (1), and the other anchoring point of the same pair is located on the ventricular side of the prosthesis (1) and protrudes toward the atrial side of this prosthesis (1), the free ends of these anchoring points (5) being located opposite one another, and the anchoring points (5) of the same pair being curved, with their concave side on the inside of this pair such that the two anchoring points (5) of the same pair of points assume the form of a "crab claw".

6. Prosthesis (1) according to claim 5, **characterized in that** said annular portion (2i) comprises six pairs of anchoring points (5).

7. Prosthesis (1) according to claim 5 or claim 6, **characterized in that** the bases of the anchoring points (5) are connected to the portions of the meshes that form at the opposite corners of these meshes in the atrial-ventricular direction of the prosthesis (1), these anchoring points protruding from these portions of the meshes.

8. Prosthesis (1) according to anyone of claims 1-7, **characterized in that** the frame (2) comprises, on the side of the base of the ventricular extensions (6), V-shaped spacers (7) connecting a lateral wire portion of one extension (6) to the lateral wire portion of the adjacent extension (6).
